# EUROPEAN PATENT APPLICATION

(11) **EP 4 260 828 A1**
(43) Date of publication of application: **18.10.2023**
(21) Application number: 21914570.3
(22) Date of filing: 29.12.2021
(51) Int. Cl.: A61B 18/12, G16H 10/60, G06F 16/906

(54) **RADIO-FREQUENCY PARAMETER CONFIGURATION METHOD, APPARATUS AND SYSTEM, AND COMPUTER-READABLE STORAGE MEDIUM**

(30) Priority: 31.12.2020 CN 202011638280
(71) Applicant: Hangzhou Broncus Medical Co., Ltd., Hangzhou, Zhejiang 310051 (CN)
(72) Inventor: XU, Hong, Hangzhou, Zhejiang 310051 (CN); CUI, Changjie, Hangzhou, Zhejiang 310051 (CN)
(74) Representative: KASTEL Patentanwälte PartG mbB
(86) International application number: PCT/CN2021/142749
(87) International publication number: WO 2022/143838

(57) **Abstract**

A radio-frequency parameter configuration method, apparatus, and system, and a computer-readable storage medium are provided. The method includes acquiring radio-frequency operation data of multiple historical radio-frequency tasks; analyzing the radio-frequency operation data, to obtain and store respective radio-frequency parameter configuration schemes corresponding to various types of ablation objects in a preset database; acquiring, in response to a triggered parameter configuration instruction, a target type of a target operation object indicated by the parameter configuration instruction; and querying the database according to the target type, to obtain a target parameter configuration scheme, and configuring the radio-frequency parameter of a target radio-frequency operation system indicated by the parameter configuration instruction. By the present invention, automatic configuration of parameters for a radio-frequency operation system based on big data is realized, with simplified parameter configuration operation, saved configuration time, and improved configuration efficiency.

## Description

### TECHNICAL FIELD

Embodiments of the present invention relate to the field of communications, and particularly to a radio-frequency parameter configuration method, apparatus, and system, and a computer-readable storage medium.

### DESCRIPTION OF THE PRIOR ART

Radio-frequency ablation (RFA) is a common technology for minimally invasive ablation of tumors. The principle of radio-frequency ablation is to use an alternating highfrequency current with a frequency of less than 30 MHz to cause high-speed oscillations and frictions of ions in tumor tissue, so the radio-frequency energy is converted into heat energy, causing coagulative necrosis of tumor cells.

Before a radio-frequency task is implemented, parameter configuration of a radio-frequency operation system is required. At present, the parameter configuration mainly depends on manual operation, and the existing radio-frequency operation system cannot independently undergo parameter configuration or give configuration reference. Therefore, the existing radio-frequency parameter configuration method requires an operator to have higher level of knowledge and operation experience, and the configuration is difficult and time consuming.

### SUMMARY OF THE DISCLOSURE

Embodiments of the present invention provide a radio-frequency parameter configuration method, apparatus, and system, and a computer-readable storage medium are provided. By the present invention, automatic configuration of parameters for a radio-frequency operation system based on big data is realized, the configuration difficulty is lowered, the parameter configuration operation is simplified, the configuration time is saved, and the configuration efficiency is improved.

In an aspect, an embodiment of the present invention provides a radio-frequency parameter configuration method, for use in a computer device. The method includes:
acquiring radio-frequency operation data of multiple historical radio-frequency tasks, wherein the radio-frequency operation data includes description data of each of the historical radio-frequency tasks, configured parameter data for each radio-frequency operation system when the corresponding historical radio-frequency task is implemented, and characteristic data of an operation object associated with each of the historical radio-frequency tasks and characteristic change data of an operation site of the associated operation object;
analyzing the radio-frequency operation data, to obtain and store radio-frequency parameter configuration schemes corresponding to various types of operation objects in a preset database;
acquiring, in response to a triggered parameter configuration instruction, a target type of a target operation object indicated by the parameter configuration instruction; and
querying the database according to the target type, to obtain a target parameter configuration scheme, and configuring the radio-frequency parameter of a target radio-frequency operation system indicated by the parameter configuration instruction according to the target parameter configuration scheme.

In an aspect, an embodiment of the present invention further provides a radio-frequency parameter configuration apparatus. The apparatus includes:
a first acquisition module, configured to acquire radio-frequency operation data of multiple historical radio-frequency tasks, wherein the radio-frequency operation data includes description data of each of the historical radio-frequency tasks, configured parameter data for each radio-frequency operation system when the historical radio-frequency task is implemented, and characteristic data of an operation object associated with each of the historical radio-frequency tasks and characteristic change data of an operation site of the associated operation object;
an analysis module, configured to analyze the radio-frequency operation data, to obtain and store respective radio-frequency parameter configuration schemes corresponding to various types of operation objects in a preset database;
a second acquisition module, configured to acquire, in response to a triggered parameter configuration instruction, a target type of a target operation object indicated by the parameter configuration instruction; and
a configuration module, configured to query the database according to the target type, to obtain a target parameter configuration scheme, and configure the radio-frequency parameter of a target radio-frequency operation system indicated by the parameter configuration instruction according to the target parameter configuration scheme.

In an aspect, an embodiment of the present invention further provides an electronic device, which includes a storage and a processor, wherein
the storage stores an executable program code; and
the processor is coupled to the storage, and configured to call the executable program code stored in the storage, and implement the radio-frequency parameter configuration method provided in the above embodiment.

In an aspect, an embodiment of the present invention further provides a radio-frequency parameter configuration system, which includes multiple radio-frequency operation systems, a parameter configuration device and a database server.

The radio-frequency operation system includes: a radio-frequency host, a radio-frequency ablation catheter, a neutral electrode and an injection pump.

The parameter configuration device is configured to implement various steps in the radio-frequency parameter configuration method provided in the above embodiment.

In an aspect, an embodiment of the present invention further provides a computer-readable storage medium storing a computer program. When the computer program is executed by a processor, the radio-frequency parameter configuration method provided in the above embodiment is implemented.

As can be known from the above embodiments of the present invention, by analyzing radio-frequency operation data of multiple historical radio-frequency tasks, respective radio-frequency parameter configuration schemes corresponding to various types of operation objects is obtained and stored in a preset database; a target parameter configuration scheme matching a target operation object indicated by an parameter configuration instruction is acquired by using the database; and the radio-frequency parameter of a target radio-frequency operation system indicated by the parameter configuration instruction is configured according to the target parameter configuration scheme. As a result, automatic configuration of system parameters based on big data is realized, with reduced configuration difficulty, simplified parameter configuration operation, saved configuration time, and improved configuration efficiency. Moreover, since the database is obtained based on massive historical operation data, the configuration result has a high reference value.

### BRIEF DESCRIPTION OF DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present invention. Other drawings can be obtained by persons of ordinary skill in the art based on these drawings without creative efforts.
FIG. 1 shows an application environment of a radio-frequency parameter configuration method provided in an embodiment of the present invention;
FIG. 2 shows a flow chart of implementing a radio-frequency parameter configuration method provided in an embodiment of the present invention;
FIG. 3 shows a flow chart of implementing a radio-frequency parameter configuration method provided in another embodiment of the present invention;
FIG. 4 is a structural schematic view of a radio-frequency parameter configuration apparatus provided in an embodiment of the present invention;
FIG. 5 is a schematic diagram showing a hardware structure of an electronic device provided in an embodiment of the present invention; and
FIG. 6 shows a structural schematic view of a radio-frequency parameter configuration system provided in an embodiment of the present invention.

### DDESCRIPTION OF EMBODIMENTS

In order to make the objects, technical solutions and advantages of the embodiments of the present invention clearer, the technical solutions according to the embodiments of the present invention will be clearly and completely described below with reference to drawings in the embodiments of the present invention. Apparently, the embodiments described are merely some embodiments, but not all of the embodiments of the present invention. All other embodiments obtained by ordinary persons skilled in the art based on the embodiments of the present invention without creative efforts shall fall within the protection scope of the present invention.

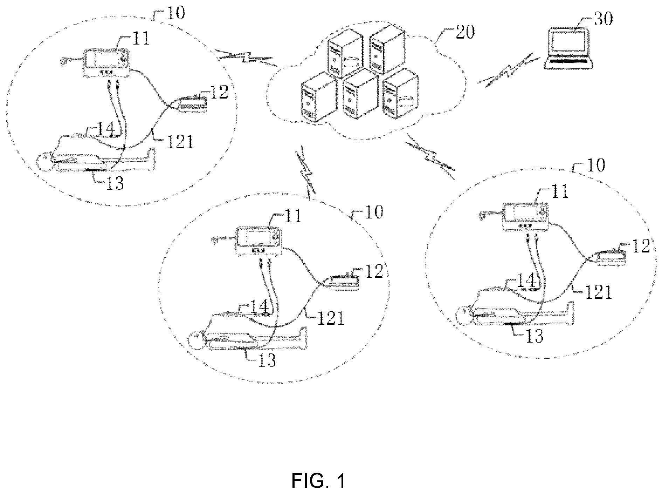

FIG. 1 shows an application scenario of a radio-frequency parameter configuration method provided in an embodiment of the present invention. The radio-frequency parameter configuration method can be implemented by a server 20 shown in FIG. 1. The server 20 may be a single cloud server, or a distributed server cluster consisting of multiple servers in the cloud. The distributed server cluster includes at least one access server group for data access, at least one distribution server group for data distribution, at least one cloud computing server group for data processing and at least one database server group for data storage (for example, database in various embodiments of the present invention). The server groups can be configured in the same place, or distributed and configured at various positions.

The access server group serves as an external interface of the distributed server cluster, and establishes, through a first gateway, data connection with the distribution server group for data exchange, to achieve data acquisition and data distribution in the radio-frequency parameter configuration method provided in various embodiments of the present invention.

The distribution server group establishes, through a second gateway, data connection with the cloud computing server group for data exchange, to achieve distribution of radio-frequency ablation data and control of data processing in the radio-frequency parameter configuration method provided in various embodiments of the present invention. The distribution server group determines, according to a real-time processing capacity of each cloud computing server, a target server for processing data sent from the access server group.

The cloud computing server group establishes, through a third gateway, data connection with the database server group, to achieve the analysis and processing of radio-frequency ablation data in the radio-frequency parameter configuration method provided in various embodiments of the present invention, and achieve the storage query and modification of data in a database in various embodiments of the present invention.

The server 20 establishes, through a wireless network or by wired connection, data connection with one or more devices in one or more radio-frequency operation systems 10 and an additional related apparatus 30, acquires, through the data connection, radio-frequency operation data of a historical radio-frequency task performed by each radio-frequency operation system 10, and processes the radio-frequency operation data by a radio-frequency parameter configuration method provided in the following embodiments. For ease of understanding, FIG. 1 merely shows 3 radio-frequency operation systems 10. The present invention is not limited thereto in practical use. The multiple radio-frequency operation systems 10 can be respectively configured at different locations, for example, in different departments of multiple entities in different regions.

It can be understood that when the server 20 only establishes data connection with some devices in the radio-frequency operation system 10, these devices can serve as an interface, to forward radio-frequency ablation data of other devices in the radio-frequency operation system 10 to the server 20.

The additional related apparatus 30 may be, for example, a medical imaging apparatus, an intelligent physical examination apparatus, a personal computer terminal of a doctor user, a server of a medical data public platform, and other radio-frequency ablation data acquisition and storage apparatus.

As shown in FIG. 1, the radio-frequency operation system 10 includes: a radio-frequency host 11, an injection pump 12, a neutral electrode 13 and a radio-frequency ablation catheter 14.

Before a radio-frequency task is implemented, the radio-frequency ablation catheter 14 configured to generate and output radio-frequency energy and an extension tube 121 of the injection pump 12 are inserted into an operation object (for example, an abnormal biological tissue), reaching an operation site. Then, the neutral electrode 13 is brought into contact with the skin surface of the operation object, and a radio-frequency current flows through the radio-frequency ablation catheter 14, such that the tissue of the operation object and the neutral electrode 13 form a circuit loop.

When the radio-frequency task is triggered, the radio-frequency ablation catheter 14 is controlled by the radio-frequency host 11 to output radio-frequency energy to the operation site by discharging, so as to implement an ablation operation on the operation site. In the meanwhile, the injection pump 12 performs an injection operation on the operation object through the extension tube 121, wherein physiological saline is injected into the operation site, to adjust the impedance and temperature of the operation site.

Moreover, before the radio-frequency task is implemented, the server 20 may configure the operation parameters of the radio-frequency operation system 10 by implementing a radio-frequency parameter configuration method provided in the following embodiments, so that each device in the radio-frequency operation system 10 may implement the radio-frequency operation according to the configured parameters.

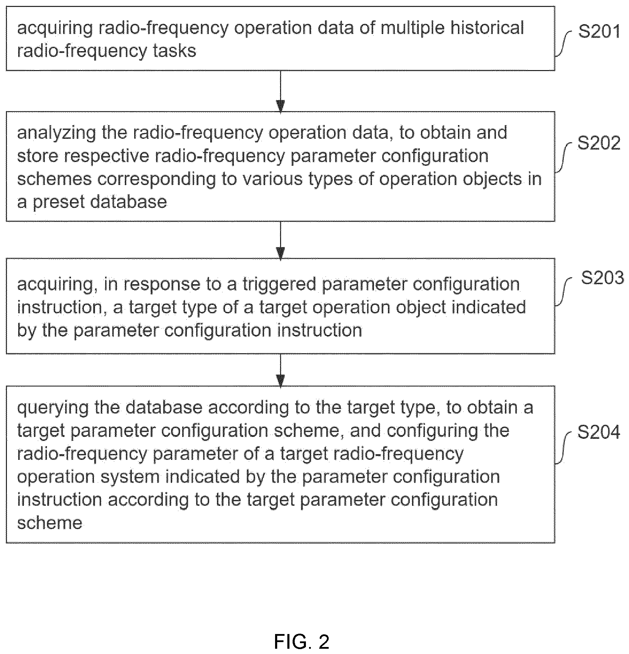

FIG. 2 shows a flow chart of implementing a radio-frequency parameter configuration method provided in an embodiment of the present invention. The method can be implemented by a computer device, such as the server 20 shown in FIG. 1, which is referred to as the server hereinafter for convenience of description. As shown in FIG. 2, the method specifically includes the following steps:

Step S201: acquiring radio-frequency operation data of multiple historical radio-frequency tasks.

Particularly, the radio-frequency operation data includes description data of each of the historical radio-frequency tasks, configured parameter data for each radio-frequency operation system when the historical radio-frequency task is implemented, and characteristic data of an operation object associated with each of the historical radio-frequency tasks and characteristic change data of an operation site of the associated operation object.

The configured parameter data for the radio-frequency operation system when the historical radio-frequency task is implemented may include, but is not limited to, for example, a radio-frequency power, a radio-frequency time, and an alarm value, called when the radio-frequency host controls the radio-frequency ablation catheter to implement the historical radio-frequency task; a liquid injection volume, a flow rate, an injection time, an alarm value of the injection pump. The radio-frequency host, the radio-frequency ablation catheter and the injection pump implement the radio-frequency task according to their respective configured parameter data.

The characteristic data of the operation object associated with the historical radio-frequency tasks may include, but is not limited to, for example, the name, age, gender, disorder, and others of the operation object.

The description data of the historical radio-frequency task may include, but is not limited to, for example, description data of the implementation time of the historical radio-frequency task and the ablation stage corresponding to the historical radio-frequency task, for example, an Nth radio-frequency task, or an nth ablation stage.

The characteristic change data of the operation site may include, but is not limited to, for example, change data of at least one of the position, the size, the shape, the volume and the area of the operation site before and after each of the associated ablation tasks is performed.

The server can acquire the configured parameter data by periodically sending a data acquisition request to a device in the radio-frequency operation system. Alternatively, the device in the radio-frequency operation system can also report the configured parameter data to the server in real time or periodically when each of the radio-frequency tasks is implemented. Alternatively, the device in the radio-frequency operation system can also report the configured parameter data of a previously implemented radio-frequency task to the server in real time or periodically.

The server can obtain the characteristic data of the operation object associated with each of the historical radio-frequency tasks according to an input operation of a user, or obtain a description file (for example, an electronic prescription) of each of the historical radio-frequency tasks from other terminals (for example, computer terminals in various medical clinics) and then extract the characteristic data of the operation object associated with each of the historical radio-frequency tasks from the description file.

Step S202: analyzing the radio-frequency operation data, to obtain and store respective radio-frequency parameter configuration schemes corresponding to various types of operation objects in a preset database.

Particularly, the operation objects can be classified first according to the description data of each of the historical radio-frequency tasks, the characteristic data of the operation object associated with each of the historical radio-frequency tasks and the characteristic change data of the operation site of the associated operation object, and a preset classification condition. Then the configured parameter data for each radio-frequency operation system when the historical radio-frequency task is implemented is classified according to the type of the operation object associated with the corresponding operation task, to obtain and store respective radio-frequency parameter configuration schemes corresponding to various types of operation objects in a preset database.

The database can be configured locally in the server, or configured in other database server.

Step S203: acquiring, in response to a triggered parameter configuration instruction, a target type of a target operation object indicated by the parameter configuration instruction.

Particularly, the parameter configuration instruction can be triggered by a user by pressing a preset physical or virtual button for triggering the parameter configuration instruction. The target type of the target operation object indicated by the parameter configuration instruction can be inputted by a user; or automatically matched according to other data inputted by the user, for example, characteristic data of the target operation object, and according to the above preset classification condition.

Step S204: querying the database according to the target type, to obtain a target parameter configuration scheme, and configuring the radio-frequency parameter of a target radio-frequency operation system indicated by the parameter configuration instruction according to the target parameter configuration scheme.

The radio-frequency parameter of the target radio-frequency operation system indicated by the parameter configuration instruction is configured for example, by sending, to a device in the target radio-frequency operation system, a configuration instruction and configured parameter data of the device in the target parameter configuration scheme, to instruct the device to configure the corresponding configured parameter data locally in the device, for being called by the device when a corresponding radio-frequency operation or radio-frequency task is implemented.

It can be understood that all parameter data of all the devices in the radio-frequency ablation system called for implementing the same ablation task may serve as an ablation parameter configuration scheme, or all parameter data of one device in the radio-frequency ablation system called for implementing one ablation task may serve as an ablation parameter configuration scheme.

In this embodiment, by analyzing radio-frequency operation data of multiple historical radio-frequency tasks, respective radio-frequency parameter configuration schemes corresponding to various types of ablation objects are obtained and stored in a preset database; by using the database, a target parameter configuration scheme corresponding to a target operation object indicated by an parameter configuration instruction is acquired; and the radio-frequency parameter of a target radio-frequency operation system indicated by the parameter configuration instruction is configured according to the target parameter configuration scheme. As a result, automatic configuration of system parameters based on big data is realized, with reduced configuration difficulty, simplified parameter configuration operation, saved configuration time, and improved configuration efficiency. Moreover, since the database is obtained based on massive historical operation data, the configuration result has a high reference value.

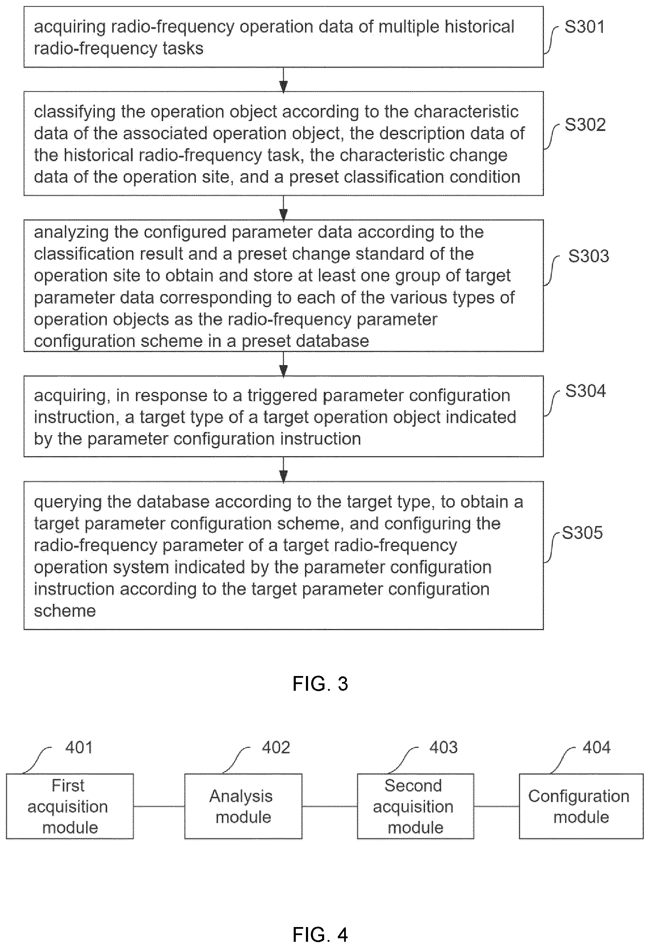

FIG. 3 shows a flow chart of implementing a radio-frequency parameter configuration method provided in another embodiment of the present invention. The method can be implemented by a computer device, such as the server 20 shown in FIG. 1, which is referred to as the server hereinafter for convenience of description. As shown in FIG. 3, the method specifically includes the following steps:

Step S301: acquiring radio-frequency operation data of multiple historical radio-frequency tasks.

Particularly, the radio-frequency operation data includes description data of each of the historical radio-frequency tasks, configured parameter data for each radio-frequency operation system when the historical radio-frequency task is implemented, and characteristic data of an operation object associated with each of the historical radio-frequency tasks and characteristic change data of an operation site of the associated operation object.

The description data of the historical radio-frequency task includes description data of the implementation time of the historical radio-frequency task, and the ablation stage corresponding to the historical radio-frequency task, for example, an Nth radio-frequency task, or an nth ablation stage.

The characteristic data of the associated operation object includes at least one of body weight, disorder before a current ablation task is implemented, blood pressure, heart beats, blood glucose, blood lipid, vital capacity, and blood oxygen saturation in a preset period of time, and age and gender of the associated operation object. The disorder includes other disorders than the disorder corresponding to the radio-frequency task, such as hypertension, high fever, asthma and so on.

The characteristic change data of the operation site includes change data of at least one of the size, the shape, the volume and the area of the operation site before and after the associated radio-frequency task is performed.

The configured parameter data for the radio-frequency operation system when the historical radio-frequency task is implemented may include, but is not limited to, for example, a radio-frequency power, a radio-frequency time, and an alarm value; a liquid injection volume, a flow rate, an injection time, an alarm value of the injection pump, called when the radio-frequency host controls the radio-frequency ablation catheter to implement the historical radio-frequency task. The radio-frequency host, the radio-frequency ablation catheter and the injection pump implement the radio-frequency task according to their respective configured parameter data.

The server can acquire the configured parameter data by periodically sending a data acquisition request to a device in the radio-frequency operation system. Alternatively, the device in the radio-frequency operation system can also report the configured parameter data in real time or periodically when each of the radio-frequency tasks is implemented to the server. Alternatively, the device in the radio-frequency operation system can also report the configured parameter data of a previously implemented radio-frequency task to the server in real time or periodically.

The server can obtain the characteristic data of the operation object associated with each of the historical radio-frequency tasks according to an input operation of a user, or obtain a description file (for example, an electronic prescription) of each of the historical radio-frequency tasks from other terminals (for example, computer terminals in various medical clinics) and then extract the characteristic data of the operation object associated with each of the historical radio-frequency tasks from the description file.

Optionally, the step of acquiring characteristic change data of an operation site of the associated operation object specifically includes:
acquiring result data of multiple imaging operations performed on the associated operation object by a medical imaging apparatus, wherein the result data includes implementation time and outputted image of each of the imaging operations; and
performing image recognition on each of the images, and comparing recognition results of the images according to the sequence of the implementation time, to obtain the characteristic change data of the operation site of the associated operation object.

The medical imaging apparatus may include, but is not limited to, an X-ray machine, and a Computed Tomography (CT) machine. Particularly, the medical imaging apparatus can report, after each imaging task is implemented or periodically, the imaging data obtained when the imaging task is performed to the server.

By the image recognition, the characteristic data of the operation site in the image is obtained, for example, at least one of the size, the shape, the area and the volume. The characteristic data in the images obtained at various implementation times is compared according to the sequence of time, to obtain the characteristic change data of the operation site before and after various imaging times. Combining the correspondence relationship between the imaging implementation time and the implementation time of the radio-frequency task, the characteristic change data of the operation site before and after various radio-frequency tasks are implemented can be obtained.

As a result, other related apparatuses such as a medical imaging apparatus are incorporated into a network for automatic analysis of the radio-frequency operation data, to achieve the automatic collection and correlation of related data, thus saving the labor cost, shortening the data acquisition time, and further improving the efficiency of data analysis.

Step S302: classifying the operation object according to the characteristic data of the associated operation object, the description data of the historical radio-frequency task, the characteristic change data of the operation site, and a preset classification condition.

Particularly, the preset classification condition is a criterion for classification. As shown in Table 1 below, according to specific contents of the radio-frequency operation data, the criterion for classification may include, but is not limited to, for example, the age, gender, disorder before an ablation task is implemented, ablation stage, characteristic range of the operation site, and range of survival time. In practical use, one or more classification conditions can be preset according to the user's custom operation.

In this way, the operation object is classified according to the preset classification condition, to distinguish various types of operation objects; and then the radio-frequency operation data is analyzed according to the classification result, such that the radio-frequency parameter configuration schemes obtained after analysis have high pertinence and reference value.

**Table 1**

| Type of operation object | Characteristic range of operation object | | Survival time | Disorder | Characteristic range of operation site | | | Ablation stage |
|---|---|---|---|---|---|---|---|---|
| | Age | Gender | | | Area | Shape | Positi on (x, y, z) | |
| Type 1 | 0-10 years | Female | 1-3 years | No | 0.5 cm | Irregular | 3, 4, 5 | 1 |
| Type 2 | 10-20 years | Male | <1 year | Gastritis | 0.1 cm | Elliptical | 10, 11, 13 | 3 |
| ...... | | | | | | | | |

As shown in Table 1 above, multiple types of operation objects are preset. Each type (for example, Type1, and Type 2) correspond to one or more classification conditions (such as age, and gender, etc.). The characteristic data of the ablation object in the radio-frequency operation data of a historical radio-frequency task, the description data of the historical radio-frequency task, and the characteristic change data of the operation site task are respectively compared with the classification conditions corresponding to various preset types, to determine a preset type corresponding to the classification condition that is most satisfied by the historical radio-frequency task. The preset type is taken as the type of the operation object. Then, the configured parameter data when the historical radio-frequency task is implemented (that is, the configured parameter data in the radio-frequency operation data) is taken as a radio-frequency parameter configuration scheme and associated with the preset type.

In Table 1 above, (x, y, z) are the position coordinates of the operation site. Optionally, a two-dimensional or three-dimensional coordinate system is established by taking a certain vertex of the whole tissue where the operation site is located as the origin. The coordinates of the center of mass of the operation site in the two-dimensional or three-dimensional coordinate system are taken as the position of the operation site. By using the coordinates, the position of the operation site can be located more accurately, thereby further improving the accuracy of the analysis results.

It can be understood that Table 1 above merely shows an example. In practical use, a related data table can have more or less content, and can also be presented in other forms.

Step S303: analyzing the configured parameter data according to the classification result and a preset change standard of the operation site to obtain and store at least one group of target parameter data corresponding to each of the various types of operation objects as the radio-frequency parameter configuration scheme in a preset database.

Particularly, the configured parameter data for each radio-frequency operation system when each of the historical radio-frequency tasks is implemented is classified according to the classification result of the operation object, to obtain at least one group of candidate parameter data corresponding to each of the various types of operation objects. Then, parameter data in the candidate parameter data where the characteristic change data of the operation site does not satisfy the preset change standard, to obtain the at least one group of target parameter data as the radio-frequency parameter configuration scheme. The finally obtained data stored in the database is shown in Table 2 below.

**Table 2**

| Type of operation object | Classification condition | Radio-frequency parameter configuration scheme |
|---|---|---|
| Type 1 | Aged 0-10 years; | First group: |
| | the operation site is reduced by 0.5 cm in the 1st ablation stage; | Power of radio-frequency ablation catheter: A |
| | | Injection volume of injection pump: B |
| | ... | |
| Type 2 | Aged 11-15 years; | First group: |
| | | Power of radio-frequency ablation catheter: C |
| | the operation site is reduced by 0.1 cm in the 2nd ablation stage; | Injection volume of injection pump: D Second group: |
| | | Power of radio-frequency ablation catheter: E |
| | ... | |
| | | Injection volume of injection pump: F |
| | | ...... |
| ...... | | |

It can be understood that Table 2 above merely shows an example. In practical use, a related data table can have more or less content, and can also be presented in other forms. It can be understood that the database is used for storing the correlation relationship or correspondence relationship between the data involved in the present invention and relevant raw data; or the database merely stores the correlation relationship or correspondence relationship and a storage link of the original data, and the raw data is stored in other database servers, to reduce the volume of the database, and improve the query speed.

The preset change standard is used to evaluate the ablation effect, and can be an absolute value, or a relative value. The change standard can correspond to at least one of the characteristic change data of the operation site, for example, a preset value to which the size of the operation site to be reduced (for example, reduced to 0.01 cm), or a value by which the area of the operation site to be reduced (for example, reduced by 0.1 cm). The type and specific value of the preset change standard can be specifically set according to the user's custom operation.

Optionally, in another embodiment of the present invention, the method further includes: setting an average of the characteristic change data of all the operation sites as the preset change standard.

Namely, when the characteristic change of the operation site reaches an average change level of all the operation sites, for example, the area change reaches an average area change level and the size change reaches an average size change level, the corresponding configured parameter data can be reserved. In this way, when a radio-frequency task is implemented by a radio-frequency operation system, all the configured parameter data of various devices configured in the system are used candidate schemes, and each candidate scheme is screened by using the ablation effect, to further improve the reference value of the analysis results.

Optionally, in another embodiment of the present invention, the method further includes analyzing normal distribution characteristics of the characteristic change data of the operation site, and setting a normal distribution characteristic value obtained after analysis as the preset change standard.

Particularly, the normal distribution is of a bell-shaped curve, having a high probability density in the middle, low probability densities at two sides, and a shape determined by the parameters µ and σ. A continuous random variable conforming a normal distribution with a mean µ and a standard deviation σ is expressed as X~N (µ, σ^2). µ refers to the central position of the curve, and generally, the maximum probability density appears near the mean. By analyzing normal distribution characteristics of the characteristic change data of the operation site, a characteristic value with the maximum probability allowing the operation site to have a normal distribution is obtained, and then it is set as the preset change standard, to further improve the reference value of the analysis results.

Optionally, to improve the reference value of the parameter configuration scheme in the database, in another embodiment of the present invention, the method further includes:
periodically acquiring physiological change data of each of the associated operation objects after each of the associated historical radio-frequency tasks is ended; and screening the target parameter data in the database according to the physiological change data.

The physiological change data can be periodically acquired by intelligent physical examination apparatus. The intelligent physical examination apparatus may be a household physical examination apparatuses, or a physical examination apparatus provided in various medical facilities, specifically for example, a smart watch, a smart bracelet, and other smart wearable devices having measuring functions for blood pressure, heart rate, and body temperature, an intelligent blood glucose detector with data transmission function, an intelligent blood pressure meter, an intelligent thermometer and so on.

The physiological change data includes at least one of body temperature, blood pressure, blood glucose, heart rate, blood lipid, vital capacity, and blood oxygen saturation.

Optionally, the step of screening the target parameter data in the database according to the physiological change data specifically includes:
obtaining a physiologically abnormal change rate according to the physiological change data; and adjusting the storage state of the target parameter data in the database according to the physiologically abnormal change rate and a preset rate.

The physiologically abnormal change rate is a proportion of the number of the same type of operation objects whose physiological data changes beyond the preset range to the total number of the type of operation objects after the associated radio-frequency task is implemented. For example, in operation objects of Type 1, the physiologically abnormal change rate is a proportion of the number of operation objects whose blood pressure value is continuously higher than a preset value for over a preset period of time in the total number of operation objects of Type 1 after the associated radio-frequency task is implemented.

The step of adjusting the storage state of the target parameter data in the database specifically includes: marking the parameter data in the target parameter data with a physiologically abnormal change rate that is greater than the preset rate as hidden, locked, or frozen in the database, such that the parameter data cannot be queried by an external device; and marking the parameter data in the target parameter data with a physiologically abnormal change rate that is not greater than the preset rate as normal, such that the parameter data can be queried by an external device.

In this way, the physiological changes of each of the operation objects are monitored by the intelligent physical examination apparatus, and the storage state of the radio-frequency parameter configuration scheme stored in the database is dynamically adjusted, such that the finally obtained radio-frequency parameter configuration scheme is more pertinent and accurate with increasing volume of data.

Step S304: acquiring, in response to a triggered parameter configuration instruction, a target type of a target operation object indicated by the parameter configuration instruction.

Particularly, the parameter configuration instruction can be triggered by a user by pressing a preset physical or virtual button for triggering the parameter configuration instruction. In response to the triggered parameter configuration instruction, description data of a radio-frequency task, characteristic data of the target operation object and characteristic data of a to-be-operated site of the target operation object indicated by the parameter configuration instruction are acquired, and then the target type is determined according to the characteristic data of the target operation object and the characteristic data of the to-be-operated site.

The description data of the radio-frequency task indicated by the parameter configuration instruction includes description data of a corresponding ablation stage.

The characteristic data of the target operation object includes at least one of body weight, disorder before a current ablation task is implemented, blood pressure in a preset period of time, heart beats, blood glucose, blood lipid, vital capacity, and blood oxygen saturation, and age and gender of the target operation object.

The characteristic data of the to-be-operated site includes at least one of the size, the shape, the volume and the area of the to-be-operated site.

The description data of the radio-frequency task, the characteristic data of the target operation object and the characteristic data of the to-be-operated site of the target operation object indicated by the parameter configuration instruction can be inputted by the user when triggering the parameter configuration instruction, or obtained by querying a present radio-frequency task information database according to identification information of the radio-frequency task inputted by the user when triggering the parameter configuration instruction. The radio-frequency task information database is configured in a database server, and configured to store the description data of the radio-frequency task to be implemented, the characteristic data of the operation object associated with the radio-frequency task to be implemented and the characteristic data of the to-be-operated site.

Step S305: querying the database according to the target type, to obtain a target parameter configuration scheme, and configuring the radio-frequency parameter of a target radio-frequency operation system indicated by the parameter configuration instruction according to the target parameter configuration scheme.

The radio-frequency parameter of the target radio-frequency operation system indicated by the parameter configuration instruction is configured for example, by sending, to a device in the target radio-frequency operation system, a configuration instruction and configured parameter data of the device in the target parameter configuration scheme, to instruct the device to configure the corresponding configured parameter data locally in the device, for being called by the device when a corresponding radio-frequency operation or radio-frequency task is implemented.

Optionally, after the step of analyzing the radio-frequency operation data, to obtain and store respective radio-frequency parameter configuration schemes corresponding to various types of operation objects in a preset database, the method further includes:
acquiring to-be-analyzed data when a parameter analysis instruction is received;
determining a type of an operation object indicated by the parameter analysis instruction according to the to-be-analyzed data and the preset classification condition;
querying, in the database, parameter data matching the determined type as reference data; and
performing feasibility analysis on the parameter data to be configured according to the reference data and outputting the analysis result.

Particularly, the parameter analysis instruction can be sent to the server by other terminals according to the user's operation. When the parameter analysis instruction is sent by other terminals to the server, the to-be-analyzed data inputted by the user or a storage link of the to-be-analyzed can also be sent to the server.

The to-be-analyzed data includes characteristic data of the operation object and operation site indicated by the parameter analysis instruction, description data of the to-be-implemented radio-frequency task, and to-be-configured parameter data.

The characteristic data of the operation object indicated by the parameter analysis instruction includes at least one of body weight, disorder before a current ablation task is implemented, blood pressure in a preset period of time, heart beats, blood glucose, blood lipid, vital capacity, and blood oxygen saturation, and age and gender of the operation object.

The characteristic data of the operation site indicated by the parameter analysis instruction includes at least one of the size, the shape, the volume and the area of the operation site.

The description data of the to-be-implemented radio-frequency task indicated by the parameter analysis instruction includes description data of a corresponding ablation stage.

The steps of determining a type of an operation object indicated by the parameter analysis instruction according to the to-be-analyzed data and the preset classification condition and querying, in the database, parameter data matching the determined type as reference data are similar to in Steps S302 and S303, can be specifically made reference to related description in Steps S302 and S303, and will not be repeated here again.

Particularly, the step of performing feasibility analysis on the parameter data to be configured according to the reference data includes comparing the reference data with the parameter data to be configured, and determining whether the difference therebetween is less than a preset difference range; if the difference is less than the preset difference range, determining the corresponding parameter data to be configured to be feasible; and if the difference is not less than the preset difference range, determining the corresponding parameter data to be configured to be unfeasible.

The analysis result includes description information indicating whether the parameter data to be configured is feasible. Further, the analysis result may further include reference data corresponding to the unfeasible parameter data to be configured.

For example, assuming that the parameter data to be configured includes injection volume and injection duration of the injection pump, power of the radio-frequency ablation catheter, and ablation duration, and according to the queried reference data, the injection volume and injection duration of the injection pump in the parameter data to be configured are feasible, and the power of the radio-frequency ablation catheter and the ablation duration are unfeasible, then the analysis result includes, in addition to the description information indicating whether the parameter data to be configured is feasible, reference data for the power of the radio-frequency ablation catheter and the ablation duration, for being referred to by a user.

In this way, by using the database, the configured parameter data for the radio-frequency task to be implemented is analyzed, so that the accuracy of parameter configuration for the radio-frequency ablation system can be improved, thereby achieving a better ablation effect.

Optionally, after the step of analyzing the radio-frequency operation data, to obtain and store radio-frequency parameter configuration schemes corresponding to various types of operation objects in a preset database, the method further includes:
acquiring to-be-queried data when a scheme query request is received;
combining multiple pieces of data in the to-be-queried data, and querying the database according to the combination result and the present classification condition, to obtain and output multiple radio-frequency parameter configuration schemes matching the to-be-queried data.

The to-be-queried data includes characteristic data of a to-be-queried operation object and a to-be-queried operation site and historical operation information indicated by the scheme query request.

In the step of combining multiple pieces of data in the to-be-queried data, for example, assuming that the to-be-queried data includes age, gender, and size of the to-be-queried site, 3 pieces of data are combined, to obtain 4 combination results, including age + gender, gender + size of the to-be-queried site, age + size of the to-be-queried site, and age + gender + size of the to-be-queried site. Then, by using the 4 combination results as a key word respectively, data corresponding to the 4 combination results is respectively compared with the preset classification condition, to obtain a classification condition that is most satisfied by the data corresponding to the 4 combination results, and take a radio-frequency parameter configuration scheme matching the type of the operation object corresponding to the classification condition that is most satisfied as the query result.

Particularly, the step of querying the database according to the combination results and the preset classification condition, to obtain and output multiple radio-frequency parameter configuration schemes matching the to-be-queried data, includes:
querying the database according to the combination results and the preset classification condition, to obtain multiple radio-frequency parameter configuration schemes matching the to-be-queried data;
screening the queried multiple radio-frequency parameter configuration schemes according to the survival time, the characteristic change data of the operation site, and a preset radio-frequency result index, to exclude a radio-frequency parameter configuration scheme that does not reach the preset radio-frequency result index; and
outputting the remaining radio-frequency parameter configuration scheme as the screening result.

The characteristic data of the to-be-queried operation object includes at least one of body weight, disorder before a current ablation task is implemented, blood pressure in a preset period of time, heart beats, blood glucose, blood lipid, vital capacity, and blood oxygen saturation, and age and gender of the to-be-queried operation object.

The characteristic data of the to-be-queried operation site includes at least one of the size, the shape, the volume and the area of the to-be-queried operation site.

The historical operation information includes: the historical number of times of performing radio-frequency operation on the to-be-queried operation object.

The preset radio-frequency result index includes reference ranges of change of the size, the shape, the volume and the area of the operation site, and reference survival time, after corresponding number of radio-frequency operations or ablation stage. For example, the radio-frequency parameter configuration scheme with a survival time of less than 2 years after one radio-frequency operation is excluded.

In this way, by presetting the radio-frequency result index, a scheme with poor radio-frequency effect is excluded from all the queried radio-frequency parameter configuration schemes, thereby further improving the reference value and pertinence of the query result.

It can be understood that the manners of acquiring various similar data involved in this embodiment are the same or similar, and can be cross-referred, unless otherwise specified. Similarly, the implementation processes of similar steps can also be cross-referred, unless otherwise specified.

In this embodiment, by analyzing radio-frequency operation data of multiple historical radio-frequency tasks, respective radio-frequency parameter configuration schemes corresponding to various types of ablation objects is obtained and stored in a preset database; a target parameter configuration scheme matching a target operation object indicated by an parameter configuration instruction is acquired by using the database; and the radio-frequency parameter of a target radio-frequency operation system indicated by the parameter configuration instruction is configured according to the target parameter configuration scheme. As a result, automatic configuration of system parameters based on big data is realized, with reduced configuration difficulty, simplified parameter configuration operation, saved configuration time, and improved configuration efficiency. Moreover, since the database is obtained based on massive historical operation data, the configuration result has a high reference value.

FIG. 4 is a structural schematic view of a radio-frequency parameter configuration apparatus provided in an embodiment of the present invention. For ease of description, only the parts relevant to the embodiments of the present invention are shown. The apparatus can be a server shown in FIG. 1, or a software module configured in the server. The apparatus includes a first acquisition module 401, an analysis module 402, a second acquisition module 403 and a configuration module 404.

The first acquisition module 401 is configured to acquire radio-frequency operation data of multiple historical radio-frequency tasks. The radio-frequency operation data includes description data of each of the historical radio-frequency tasks, configured parameter data for each radio-frequency operation system when the historical radio-frequency task is implemented, and characteristic data of an operation object associated with each of the historical radio-frequency tasks and characteristic change data of an operation site of the associated operation object.

The analysis module 402 is configured to analyze the radio-frequency operation data, to obtain and store respective radio-frequency parameter configuration schemes corresponding to various types of operation objects in a preset database.

The second acquisition module 403 is configured to acquire, in response to a triggered parameter configuration instruction, a target type of a target operation object indicated by the parameter configuration instruction.

The configuration module 404 is configured to query the database according to the target type, to obtain a target parameter configuration scheme, and configure the radio-frequency parameter of a target radio-frequency operation system indicated by the parameter configuration instruction according to the target parameter configuration scheme.

Optionally, the analysis module 402 is further configured to classify the operation object according to the characteristic data of the associated operation object, the description data of the historical radio-frequency task, the characteristic change data of the operation site, and a preset classification condition; and analyze the configured parameter data according to the classification result and a preset change standard of the operation site, to obtain at least one group of target parameter data corresponding to each of the various types of operation objects as the radio-frequency parameter configuration scheme.

The analysis module 402 is further configured to classify the configured parameter data according to the classification result, to obtain at least one group of candidate parameter data corresponding to the various types of operation objects;

exclude parameter data in the candidate parameter data where the characteristic change data of the operation site does not reach the preset change standard, to obtain the at least one group of target parameter data as the radio-frequency parameter configuration scheme.

The analysis module 402 is further configured to set an average of the characteristic change data of all the ablation sites as the preset change standard.

The first acquisition module 401 is further configured to periodically acquire physiological change data of each of the associated operation objects after each of the associated historical radio-frequency tasks is ended.

The analysis module 402 is further configured to screen the target parameter data in the database according to the physiological change data.

The analysis module 402 is further configured to obtain a physiologically abnormal change rate according to the physiological change data, wherein the physiological change data includes at least one of body temperature, blood pressure, blood glucose, heart rate, blood lipid, vital capacity, and blood oxygen saturation; and adjust the storage state of the target parameter data in the database according to the physiologically abnormal change rate and a preset rate.

The first acquisition module 401 is further configured to acquire result data of multiple imaging operations performed on the associated operation object by a medical imaging apparatus, wherein the result data includes implementation time and outputted image of each of the imaging operations.

The analysis module 402 is further configured to perform image recognition on each of the images, and compare the recognition results of the images according to the sequence of the implementation time, to obtain the characteristic change data of the operation site of the associated operation object.

The analysis module 402 is further configured to acquire to-be-analyzed data when a parameter analysis instruction is received, wherein the to-be-analyzed data includes characteristic data of an operation object and an operation site indicated by the parameter analysis instruction, description data of a to-be-implemented radio-frequency task, and parameter data to be configured,;
determine a type of the operation object indicated by the parameter analysis instruction according to the to-be-analyzed data;
query, in the database, parameter data matching the determined type as reference data; and
perform feasibility analysis on the parameter data to be configured according to the reference data and output the analysis result.

Further, the characteristic data of the associated operation object includes at least one of body weight, disorder before a current ablation task is implemented, blood pressure in a preset period of time, heart beats, blood glucose, blood lipid, vital capacity, and blood oxygen saturation, and age and gender of the associated operation object;

The description data of the historical radio-frequency task includes description data of the implementation time of the historical radio-frequency task and the ablation stage corresponding to the historical radio-frequency task.

The characteristic change data of the operation site includes change data of at least one of the size, the shape, the volume and the area of the operation site before and after the associated radio-frequency task is performed.

The second acquisition module 403 is further configured to acquire description data of the radio-frequency task, characteristic data of the target operation object and characteristic data of a to-be-operated site of the target operation object, indicated by the parameter configuration instruction; and determine the target type according to the characteristic data of the target operation object and the characteristic data of the to-be-operated site.

Further, the description data of the radio-frequency task indicated by the parameter configuration instruction includes description data of a corresponding ablation stage.

The characteristic data of the target operation object includes at least one of body weight, disorder before a current ablation task is implemented, blood pressure in a preset period of time, heart beats, blood glucose, blood lipid, vital capacity, and blood oxygen saturation, and age and gender of the target operation object.

The characteristic data of the to-be-operated site includes at least one of the size, the shape, the volume and the area of the to-be-operated site.

The analysis module 402 is further configured to acquire to-be-queried data when a scheme query request is received, wherein the to-be-queried data includes characteristic data and historical operation information of a to-be-queried operation object and a to-be-queried operation site indicated by the scheme query request; and combine multiple pieces of data in the to-be-queried data, and query the database according to the combination result and the present classification condition, to obtain and output multiple radio-frequency parameter configuration schemes matching the to-be-queried data.

The analysis module 402 is further configured to query the database according to the combination result and the present classification condition, to obtain multiple radio-frequency parameter configuration schemes matching the to-be-queried data; and screen the queried multiple radio-frequency parameter configuration schemes according to the survival time, the characteristic change data of the operation site, and a preset radio-frequency result index, and output the screening result.

The characteristic data of the to-be-queried operation object includes at least one of body weight, disorder before a current ablation task is implemented, blood pressure in a preset period of time, heart beats, blood glucose, blood lipid, vital capacity, and blood oxygen saturation, and age and gender of the to-be-queried operation object.

The characteristic data of the to-be-queried operation site includes at least one of the size, the shape, the volume and the area of the to-be-queried operation site.

The historical operation information includes: the historical number of times of performing radio-frequency operation on the to-be-queried operation object.

The preset radio-frequency result index includes reference ranges of change of the size, the shape, the volume and the area of the operation site, and reference survival time, after corresponding number of radio-frequency operations.

The specific process for the above modules to implement their respective functions can be made reference to the relevant description in the embodiments shown in FIG. 2 and FIG. 3, and will not be repeated here again.

In this embodiment, by analyzing radio-frequency operation data of multiple historical radio-frequency tasks, respective radio-frequency parameter configuration schemes corresponding to various types of ablation objects is obtained and stored in a preset database; a target parameter configuration scheme matching a target operation object indicated by an parameter configuration instruction is acquired by using the database; and the radio-frequency parameter of a target radio-frequency operation system indicated by the parameter configuration instruction is configured according to the target parameter configuration scheme. As a result, automatic configuration of system parameters based on big data is realized, with reduced configuration difficulty, simplified parameter configuration operation, saved configuration time, and improved configuration efficiency. Moreover, since the database is obtained based on massive historical operation data, the configuration result has a high reference value.

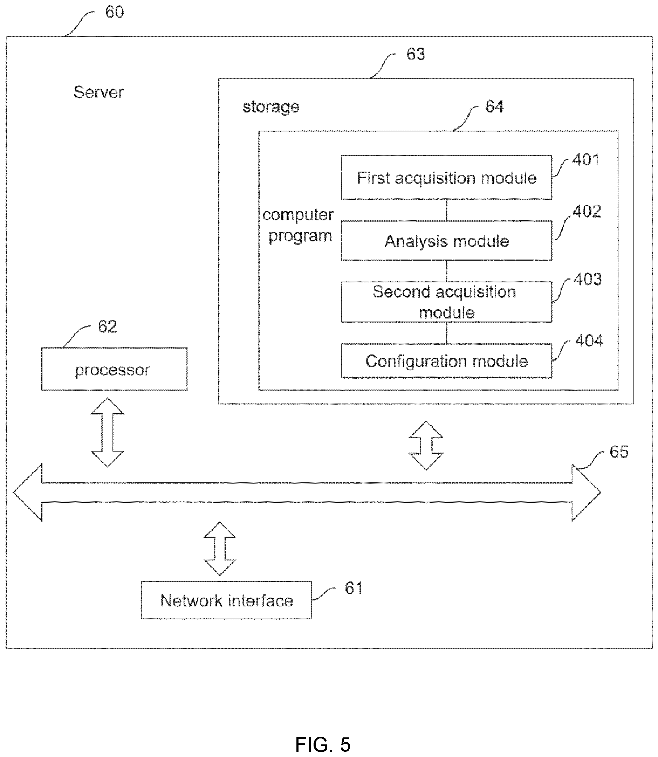

FIG. 5 is a schematic diagram showing a hardware structure of an electronic device provided in an embodiment of the present invention. As shown in FIG. 5, the electronic device 60 includes a network interface 61, a processor 62, a storage 63, a computer program 64 stored on the storage 63 and running on the processor 62, and a system bus 65. The system bus 65 is connected to the network interface 61, the processor 62 and the storage 63. When the computer program 64 is executed by the processor 62, the radio-frequency parameter configuration method according to various embodiments above is implemented.

The network interface 61 is configured to communicate with other electronic devices.

Exemplarily, the processor 62 may be a central processing unit (CPU) or other general-purpose processors, a digital signal processor (DSP), an application specific integrated circuit (ASIC), a field-programmable gate array (FPGA) or other programmable logic devices, a discrete gate or transistor logic device, a discrete hardware assembly, and others. A general-purpose processor can be a microprocessor or any conventional processors.

Exemplarily, the storage 63 is, for example, a hard drive storage, a non-transitory storage, a non-volatile storage (such as flash memory or other storages that are used to form solid-state drives and are electronically programmable to confine the deletion, etc.), and a volatile storage (such as static or dynamic random access storage), which is not limited in the embodiments of the present invention. The storage 63 can include both an internal storage unit of the electronic device 60 and an external storage device. The storage 63 is configured to store the computer program and other programs and data required by the electronic device 60. The storage 63 may also be configured to temporarily store data that have been outputted or will be outputted.

Exemplarily, the computer program 64 can be divided into one or more modules/units, stored in the storage 63 and implemented by the processor 62, to accomplish the present invention. The one or more modules/units can be a series of computer program instruction segments capable of implementing particular functions, and configured to describe an execution process of the computer program 64 by the electronic device 60. For example, the computer program 64 can be divided into a first acquisition module 401, an analysis module 402, a second acquisition module 403 and a configuration module 404. The functions of the modules are as described below.

The first acquisition module 401 is configured to acquire radio-frequency operation data of multiple historical radio-frequency tasks. The radio-frequency operation data includes description data of each of the historical radio-frequency tasks, configured parameter data for each radio-frequency operation system when the historical radio-frequency task is implemented, and characteristic data of an operation object associated with each of the historical radio-frequency tasks and characteristic change data of an operation site of the associated operation object.

The analysis module 402 is configured to analyze the radio-frequency operation data, to obtain and store respective radio-frequency parameter configuration schemes corresponding to various types of operation objects in a preset database.

The second acquisition module 403 is configured to acquire, in response to a triggered parameter configuration instruction, a target type of a target operation object indicated by the parameter configuration instruction.

The configuration module 404 is configured to query the database according to the target type, to obtain a target parameter configuration scheme, and configure the radio-frequency parameter of a target radio-frequency operation system indicated by the parameter configuration instruction according to the target parameter configuration scheme.

Optionally, the computer program 64 can be further divided to include other modules in the radio-frequency parameter configuration apparatus.

The specific process for the above function modules to implement their functions can be made reference to the relevant description in the embodiment shown in FIG. 4, and will not be repeated here again.

Further, the storage 63 further stores a device driver program, which may be a network and interface driver program.

It can be understood by those skilled in the art that FIG. 5 merely shows an example of the electronic device 60, which does not constitute a limitation on the electronic device 60. In practical use, more or fewer components, or a combination of some components, or different components can be included. For example, the electronic device 60 may further include an input/output device (such as a keyboard, a microphone, a camera, a loudspeaker, and a display screen, etc.).

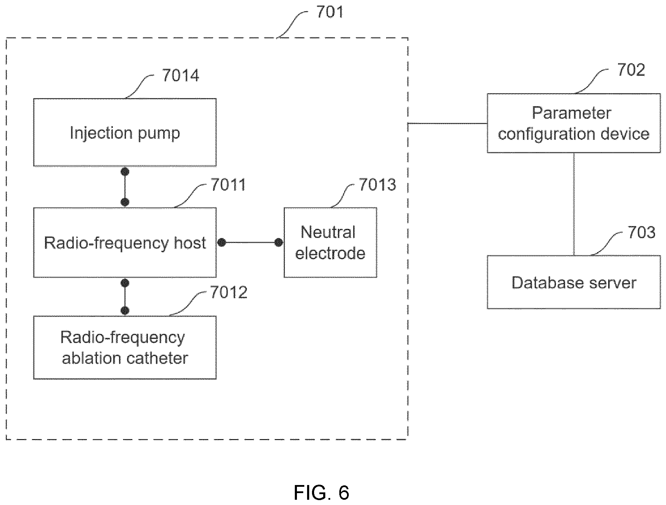

Further, as shown in FIG. 6, an embodiment of the present invention further provides a radio-frequency parameter configuration system. For ease of description, only the parts relevant to the embodiments of the present invention are shown. The radio-frequency parameter configuration system includes multiple radio-frequency operation systems 701 (for ease of understanding, only one is shown in FIG. 6), a parameter configuration device 702 and a database server 703.

The radio-frequency operation system 701 includes: a radio-frequency host 7011, a radio-frequency ablation catheter 7012, a neutral electrode 7013 and an injection pump 7014. The database server 703 may be a single server, or a distributed server cluster consisting of multiple servers.

The parameter configuration device 702 is configured to implement various steps in the radio-frequency parameter configuration method provided in the embodiments shown in FIGs. 2 and 3.

The database server 703 is configured to store various database involved in the embodiments shown in FIGs. 2 and 3, and provide data query service based on these database to the parameter configuration device 702 and various devices in the radio-frequency operation system 701.

The specific processes for the radio-frequency operation system 701, the parameter configuration device 702 and the database server 703 to implement their respective functions can be made reference to the relevant description in the embodiments shown in FIGs. 1 to 5, and will not be repeated here again.

In this embodiment, by analyzing radio-frequency operation data of multiple historical radio-frequency tasks, respective radio-frequency parameter configuration schemes corresponding to various types of ablation objects is obtained and stored in a preset database; a target parameter configuration scheme matching a target operation object indicated by an parameter configuration instruction is acquired by using the database; and the radio-frequency parameter of a target radio-frequency operation system indicated by the parameter configuration instruction is configured according to the target parameter configuration scheme. As a result, automatic configuration of system parameters based on big data is realized, with reduced configuration difficulty, simplified parameter configuration operation, saved configuration time, and improved configuration efficiency. Moreover, since the database is obtained based on massive historical operation data, the configuration result has a high reference value.

Further, an embodiment of the present invention further provides a computer-readable storage medium, which can be provided in the electronic device in each of the above embodiments, and may be a storage in a storage and processing circuit 300 in the embodiment shown in FIG. 5. A computer program is stored in the computer-readable storage medium. When the program is executed by a processor, the radio-frequency parameter configuration method described in the embodiments above is implemented. Further, the computer-readable storage medium may also be a U disk, a movable hard disk, a read-only memory (ROM), RAM, a magnetic disk, an optical disc and other media that can store program codes.

In the embodiments provided in the present invention, it can be understood that the disclosed apparatus and method may be implemented in other ways. For example, the apparatus embodiments described above are merely illustrative. For example, the division of the modules is merely a division of logical functions, and there may be additional divisions in actual implementation. For example, multiple modules or components may be combined or integrated into another system, or some features may be omitted, or not performed. Alternatively, the couplings or direct couplings or communicative connections shown or discussed with respect to one another may be indirect couplings or communicative connections via some interfaces, devices or modules, and may be electrical, mechanical or otherwise.

The modules described as separate components may or may not be physically separate, and the components shown as modules may or may not be physical modules, i.e. they may be located in one place, or may be distributed over a plurality of network modules. Some or all of the modules may be selected to achieve the objectives of the solution of the present embodiment according to practical requirements.

In addition, the functional modules in the various embodiments of the present invention may be integrated into one processing module, may be physically separate from each other or may be may be one module integrated by two or more modules. The integrated modules described above can be implemented either in the form of hardware, or software functional modules.

The integrated module, if implemented in the form of a software program module and sold or used as a stand-alone product, may be stored in a computer-readable storage medium. Based on such an understanding, the essence of the technical solution of present invention or part thereof contributing to the related art, or all or part of the technical solution can be implemented by a software product, The computer software product is stored in a readable storage medium, and includes several instructions executable by a computer device (for example, personnel computer, a server, or a network device) to perform all or some of the steps in the methods according to various embodiments of the present invention. The readable storage medium includes: a U disk, a movable hard disk, ROM, RAM, a magnetic disk, an optical disc and other media that can store program codes.

It should be noted that for ease of description of the above method embodiments, they are all described as a series of actions. However, it is to be known by those skilled in the art that the present invention is not limited to the sequence of actions described, because in the present invention, some steps can be done in other sequences or simultaneously. Further, it is to be known by those skilled in the art that embodiments described in the specification are all preferred embodiments, and the actions and modules involved therein are not necessarily required for the present invention.

In the embodiments described above, emphasis has been placed on the description of various embodiments. Parts of an embodiment that are not described in detail may be found in the description of other embodiments.

The radio-frequency parameter configuration method, apparatus, and system, and the computer-readable storage medium provided in the present invention are described above. Changes can be made to the specific implementation and the scope of application by those skilled in the art according to the idea of the embodiments of the present invention. Therefore, the disclosure of this specification should not be construed as a limitation of the present invention.

## Claims

1. A configuration method for a radio-frequency parameter, for use in a computer device, comprising steps of:
acquiring radio-frequency operation data of a plurality of historical radio-frequency tasks, wherein the radio-frequency operation data comprises description data of each of the historical radio-frequency tasks, configured parameter data for each radio-frequency operation system when the corresponding historical radio-frequency task is implemented, and characteristic data of an operation object associated with each of the historical radio-frequency tasks and characteristic change data of an operation site of the associated operation object;
analyzing the radio-frequency operation data, to obtain and store respective radio-frequency parameter configuration schemes corresponding to various types of ablation objects in a preset database;
acquiring, in response to a triggered parameter configuration instruction, a target type of a target operation object indicated by the parameter configuration instruction; and
querying the database according to the target type, to obtain a target parameter configuration scheme, and configuring the radio-frequency parameter of a target radio-frequency operation system indicated by the parameter configuration instruction according to the target parameter configuration scheme.

2. The method of claim 1, wherein the step of analyzing the radio-frequency operation data to obtain respective radio-frequency parameter configuration schemes corresponding to various types of ablation objects comprises:
classifying the operation object according to the characteristic data of the associated operation object, the description data of the historical radio-frequency task, the characteristic change data of the operation site, and a preset classification condition; and
analyzing the configured parameter data according to a classification result and a preset change standard of the operation site, to obtain at least one group of target parameter data corresponding to each of the various types of operation objects as the radio-frequency parameter configuration scheme.

3. The method of claim 2, wherein the step of analyzing the configured parameter data according to a classification result and a preset change standard of the operation site to obtain at least one group of target parameter data corresponding to each of the various types of operation objects as the radio-frequency parameter configuration scheme, comprises:
classifying the configured parameter data according to the classification result, to obtain at least one group of candidate parameter data corresponding to each of the various types of operation objects;
excluding parameter data in the candidate parameter data where the characteristic change data of the operation site does not reach the preset change standard, to obtain the at least one group of target parameter data as the radio-frequency parameter configuration scheme.

4. The method of claim 3, further comprising:
setting an average of the characteristic change data of each of the operation sites as the preset change standard.

5. The method of claim 3, further comprising:
analyzing normal distribution characteristics of the characteristic change data of the operation site, and setting a normal distribution characteristic value obtained after analysis as the preset change standard.

6. The method of claim 2, further comprising:
periodically acquiring physiological change data of each of the associated operation objects after each of the associated historical radio-frequency tasks is ended; and
screening the target parameter data in the database according to the physiological change data.

7. The method of claim 6, wherein the step of screening the target parameter data in the database according to the physiological change data comprises:
obtaining a physiologically abnormal change rate according to the physiological change data, wherein the physiological change data comprises at least one of body temperature, blood pressure, blood glucose, heart rate, blood lipid, vital capacity, and blood oxygen saturation; and adjusting a storage state of the target parameter data in the database according to the physiologically abnormal change rate and a preset rate.

8. The method of claim 1, wherein the step of acquiring characteristic change data of an operation site of the associated operation object comprises:
acquiring result data of a plurality of imaging operations performed on the associated operation object by a medical imaging apparatus, wherein the result data comprises implementation time and outputted image of each of the imaging operations; and
performing image recognition on each of the images, and comparing recognition results of the images according to the sequence of the implementation time, to obtain the characteristic change data of the operation site of the associated operation object.

9. The method of claim 1, further comprising:
acquiring to-be-analyzed data when a parameter analysis instruction is received, wherein the to-be-analyzed data comprises characteristic data of an operation object and an operation site indicated by the parameter analysis instruction, description data of an ablation task to be implemented, and parameter data to be configured;
determining a type of the operation object indicated by the parameter analysis instruction according to the to-be-analyzed data;
querying, in the database, parameter data matching the determined type as reference data; and
performing feasibility analysis on the parameter data to be configured according to the reference data and outputting an analysis result.

10. The method of any one of claims 1 to 9, wherein
the characteristic data of the operation object comprises at least one of body weight, disorder before a current ablation task is implemented, blood pressure in a preset period of time, heart beats, blood glucose, blood lipid, vital capacity, and blood oxygen saturation, as well as age and gender of the operation object;
the description data of the historical radio-frequency task comprises description data of the implementation time of the historical radio-frequency task, and the ablation stage corresponding to the historical radio-frequency task; and
the characteristic change data of the operation site comprises change data of at least one of the size, the shape, the volume and the area of the operation site before and after an associated radio-frequency task is performed.

11. The method of claim 1, wherein the step of acquiring a target type of a target operation object indicated by the parameter configuration instruction comprises:
acquiring description data of a radio-frequency task, characteristic data of the target operation object and characteristic data of a to-be-operated site of the target operation object indicated by the parameter configuration instruction; and
determining the target type according to the characteristic data of the target operation object and the characteristic data of the to-be-operated site.

12. The method of claim 11, wherein
the description data of the radio-frequency task indicated by the parameter configuration instruction comprises description data of a corresponding ablation stage,
the characteristic data of the target operation object comprises at least one of body weight, disorder before a current ablation task is implemented, blood pressure in a preset period of time, heart beats, blood glucose, blood lipid, vital capacity, and blood oxygen saturation, as well as age and gender of the target operation object; and
the characteristic data of the to-be-operated site comprises at least one of the size, the shape, the volume and the area of the to-be-operated site.

13. The method of claim 10, further comprising:
acquiring to-be-queried data when a scheme query request is received, wherein the to-be-queried data comprises characteristic data and historical operation information of a to-be-queried operation object and a to-be-queried operation site indicated by the scheme query request; and
combining a plurality of pieces of data in the to-be-queried data, and querying the database according to the combination result and the present classification condition, to obtain and output a plurality of radio-frequency parameter configuration schemes matching the to-be-queried data.

14. The method of claim 13, wherein the step of querying the database according to the combination result and the present classification condition to obtain and output a plurality of radio-frequency parameter configuration schemes matching the to-be-queried data comprises
querying the database according to the combination result and the present classification condition, to obtain a plurality of radio-frequency parameter configuration schemes matching the to-be-queried data; and
screening the queried plurality of radio-frequency parameter configuration schemes according to survival time, characteristic change data of the operation site, and a preset radio-frequency result index, and outputting the screening result.

15. The method of claim 13 or 14, wherein
the characteristic data of the to-be-queried operation object comprises at least one of body weight, disorder before a current ablation task is implemented, and blood pressure, heart beats, blood glucose, blood lipid, vital capacity, and blood oxygen saturation in a preset period of time, and age and gender of the to-be-queried operation object;
the characteristic data of the to-be-queried operation site comprises at least one of the size, the shape, the volume and the area of the to-be-queried operation site;
the historical operation information comprises: the historical number of times of performing radio-frequency operation on the to-be-queried operation object; and
the preset radio-frequency result index comprises reference ranges of change of the size, the shape, the volume and the area of the operation site, and reference survival time, after corresponding number of radio-frequency operations.

16. A radio-frequency parameter configuration apparatus, comprising:
a first acquisition module, configured to acquire radio-frequency operation data of multiple historical radio-frequency tasks, wherein the radio-frequency operation data comprises description data of each of the historical radio-frequency tasks, configured parameter data for each radio-frequency operation system when the historical radio-frequency task is implemented, and characteristic data of an operation object associated with each of the historical radio-frequency tasks and characteristic change data of an operation site of the associated operation object;
an analysis module, configured to analyze the radio-frequency operation data, to obtain and store respective radio-frequency parameter configuration schemes corresponding to various types of ablation objects in a preset database;
a second acquisition module, configured to acquire, in response to a triggered parameter configuration instruction, a target type of a target operation object indicated by the parameter configuration instruction; and
a configuration module, configured to query the database according to the target type, to obtain a target parameter configuration scheme, and configure the radio-frequency parameter of a target radio-frequency operation system indicated by the parameter configuration instruction according to the target parameter configuration scheme.

17. An electronic device, comprising:
a storage and a processor, wherein
the storage stores an executable program code; and
the processor is coupled to the storage, and configured to call the executable program code stored in the storage, and implement the radio-frequency parameter configuration method of any one of claims 1 to 15.

18. A radio-frequency parameter configuration system, comprising multiple radio-frequency operation systems, a parameter configuration device and a database server, wherein
the radio-frequency operation system comprises a radio-frequency host, a radio-frequency ablation catheter, a neutral electrode and an injection pump; and
the parameter configuration device is configured to implement various steps in the radio-frequency parameter configuration method of any one of claims 1 to 15.

19. A computer-readable storage medium, storing a computer program, wherein when the computer program is executed by a processor, the radio-frequency parameter configuration method of any one of claims 1 to 15 is implemented.
